# EUROPÄISCHE PATENTSCHRIFT

(11) **EP 1 438 025 B1**
(45) Veröffentlichungstag und Bekanntmachung des Hinweises auf die Patenterteilung: **05.12.2007**
(21) Anmeldenummer: 02782923.3
(22) Anmeldetag: 16.10.2002
(51) Int. Cl.: A61K 9/16, A61K 9/50

(54) **VERFAHREN ZUR HERSTELLUNG VON WIRKSTOFFHALTIGEN PELLETS**
METHOD FOR PRODUCTION OF ACTIVE INGREDIENT-CONTAINING PELLETS
PROCEDE DE PRODUCTION DE GRANULES CONTENANT UN PRINCIPE ACTIF

(30) Priorität: 22.10.2001 DE 10151290
(43) Veröffentlichungstag der Anmeldung: 21.07.2004
(73) Patentinhaber: Evonik Röhm GmbH, 64293 Darmstadt (DE)
(72) Erfinder: PETEREIT, Hans-Ulrich, 64291 Darmstadt (DE); BERGMANN, Günter, 63538 Gross-Krotzenburg (DE); LEHMANN, Klaus, 64380 Rossdorf (DE)
(86) Internationale Anmeldenummer: PCT/EP2002/011556
(87) Internationale Veröffentlichungsnummer: WO 2003/035038

(56) Entgegenhaltungen:
- EP-A- 0 629 398
- WO-A-92/01443
- DE-A- 4 232 978
- DE-A- 10 013 029
- GRAF E ET AL: "[Microencapsulation by droplet dispersion]" PHARMAZIE IN UNSERER ZEIT. GERMANY, WEST MAY 1984, Bd. 13, Nr. 3, Mai 1984 (1984-05), Seiten 71-82, XP001122145 ISSN: 0048-3664 in der Anmeldung erwähnt

## Beschreibung

Die Erfindung betrifft ein Verfahren zur Herstellung von wirkstoffhaltigen Pellets sowie dadurch herstellbare Pellets mit einem mehrschichtigem Aufbau und deren Verwendung.

### Stand der Technik

DE 100 13 029 beschreibt die Verwendung einer mehrschichtigen Arzneiform, die im wesentlichen aufgebaut ist aus einem Kern mit einem pharmazeutischen Wirkstoff, einem inneren Überzug aus einem Copolymeren oder einer Mischung von Copolymeren, die sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen und einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylatMonomeren mit einer anionischen Gruppe im Alkylrest zusammensetzt.

Graf, E. und Bothe W. beschreiben in "Mikroverkapselung durch Zertropfen". (Pharmazie in unserer Zeit, 1984, Nr. 3, S. 71 bis 82) insbesondere die Mikroverkapselung von Arzneistoffen durch Zertropfen von Flüssigkeitsstrahlen. Die Herstellung von Mikrokapseln wird unter anderem am Beispiel von Methacrylat-Copolymeren mit anionischen Resten (EUDRAGIT® S100) aufgezeigt. Dazu wird EUDRAGIT® S100 in einer Mischung aus Ethanol, Aceton und Isopropanol gelöst und zusätzlich ein Wasseranteil eingearbeitet und zur Verkapselung von Olivenöl verwendet. Als Fällbad wird angesäuertes Wasser mit pH 4,0 eingesetzt. Die erhalten Kapseln sind sehr dünnwandig und vermögen nicht das Öl zu halten. Die Mikroverkapselung von Ölen mit EUDRAGIT® zu freifließenden Pulvern wird als nicht möglich erachtet. Allerdings wird die Verwendung solcher Kapseln in wässrigen Liquida als denkbar betrachtet.

### Aufgabe und Lösung

Die Herstellung von Arzneiformen wie Tabletten oder Pellets durch das Überziehen wirkstoffhaltiger Kerne mit einem polymeren Überzugsmittel ist hinreichend bekannt. Es sollte ein alternatives Verfahren zur Herstellung von wirkstoffhaltigen Pellets bereitgestellt werden, wobei der Einsatz von organischen Lösungsmitteln vermieden werden sollte.

### Die Aufgabe wird gelöst durch ein

Verfahren zur Herstellung von wirkstoffhaltigen Pellets
durch die Schritte
a) Suspendieren oder Lösen eines pharmazeutischen Wirkstoffs in einer Dispersion, die ein kationisches Copolymer enthält,
b) Zertropfen der Dispersion, enthaltend den pharmazeutischen Wirkstoff und das Copolymer in eine wässrige Polymerlösung, die ein mit dem kationischen Copolymer unverträgliches anionisches Copolymer enthält, wodurch es zu einer Ausfällung wirkstoffhaltiger Pellets kommt
c) Abtrennen der wirkstoffhaltigen Pellets aus der wässrigen Polymerlösung und anschließendes Trocknen der Pellets

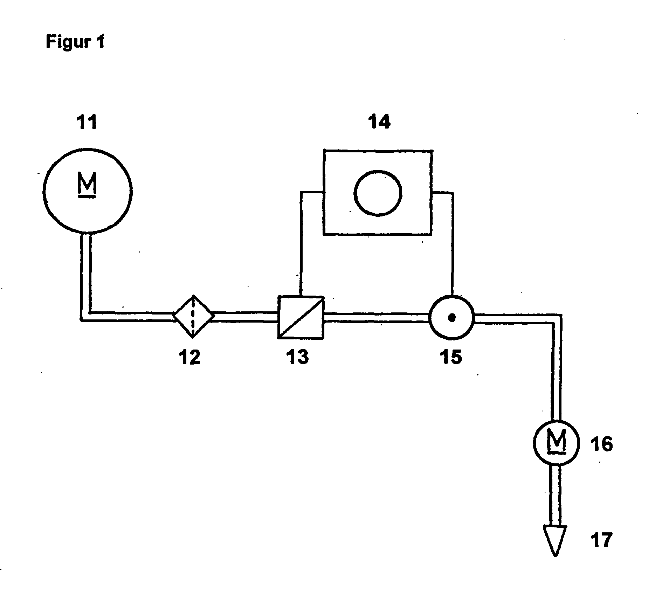

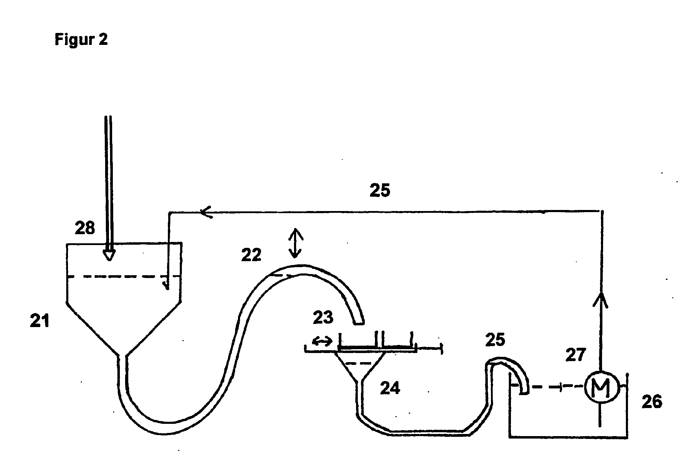

Die Erfindung wird durch die Figuren 1/2 und 2/2 verdeutlicht, ist aber nicht auf diese Darstellung beschränkt.

Fig. 1/2: Schematischer Aufbau einer Tropfapparatur zum Zertropfen der wirkstoffhaltigen Dispersion.

Bezugszeichen:
11 = Förderpumpe, Drucktopf, Schlauchpumpe oder Exzenterschneckenpumpe (z. B. Typ 6.2, Fa. Wangen GmbH, 7988 Wangen, Deutschland), M = Motor
12 = Feinstfilter 15 µm (z. B. Typ FW, Fa. B.E.S.T, 6000 Frankfurt am Main 60, Deutschland)
13 = Nadelventil mit regulierbarem Stellmotor (z. B. Typ SS, Fa. B.E.S.T)
14 = PID-Regler mit digitaler Druckanzeige (z. B. Typ 810, Fa. Eurotherm, 62500 Limburg, Deutschland)
15 = Druckaufnehmer 0 bis 1,67 bar (z. B. Typ AB, Fa, Frey, 80000 München, Deutschland)
16 = Vibrationsantrieb (z. B. Typ DR 40, Fa. Retsch GmbH, 5657 Haan 1),M = Motor
17 = Düse mit wechselbarem Einsatz (z. B. Typ WAI, Fa. Walther, 6500 Wuppertal)

Fig. 2/2: Schematischer Aufbau des Fällungsbades mit der Lösung des anionischen Methacrylat-Copolymeren
21 = Auffangbehälter für Fällungsbadflüssigkeit
22 = Flexibler Schlauch zum Abführen gefällter Pellets. Erlaubt die Einstellung des Flüssigkeitsspiegels im Auffangbehälter nach dem Siphonprinzip
23 = Wechselbare Siebe zum Abtrennen der ausgefällten Pellets
24 = Trichter zum Auffangen der geklärten Fällungsflüssigkeit
25 = Schlauch
26 = Vorratsbehälter für Fällungsbadflüssigkeit
27 = Schlauchpumpe zur Erzeugung einer konstanten Strömung (M = Motor der Schlauchpumpe)
28 = Düse der Tropfapparatur (entspricht Fig.1/2 (17))

### Ausführung der Erfindung

Die Erfindung betrifft ein Verfahren zur Herstellung von wirkstoffhaltigen Pellets

durch die Schritte
a) Suspendieren oder Lösen eines pharmazeutischen Wirkstoffs in einer Dispersion, die ein kationisches Copolymer enthält,
b) Zertropfen der Dispersion, enthaltend den pharmazeutischen Wirkstoff und das Copolymer in eine wässrige Polymerlösung, die ein mit dem kationischen Copolymer unverträgliches anionisches Copolymer enthält, wodurch es zu einer Ausfällung wirkstoffhaltiger Pellets kommt
c) Abtrennen der wirkstoffhaltigen Pellets aus der wässrigen Polymerlösung und anschließendes Trocknen der Pellets

### Verfahrensschritt a)

Suspendieren oder Lösen eines pharmazeutischen Wirkstoffs in einer Dispersion, die ein kationisches Copolymer enthält. Diese kann vereinfacht auch als Tropfflüssigkeit bezeichnet werden.

### Kationisches Copolymer

Geeignet ist ein kationisches Copolymer, das aus radikalisch polymerisierten Einheiten von C₁- bis C₄- Alkylestern der Acryl- oder Methacrylsäure und Einheiten von (Meth)acrylat-Monomeren mit tertiären oder quaternären Amino- bzw. Ammoniumgruppen besteht.

Das Copolymer setzt sich aus 30 bis 80 Gew.-% radikalisch polymerisierten C₁-bis C₄-Alkylestern der Acryl- oder der Methacrylsäure und 70 bis 20 Gew.-% (Meth)acrylat-Monomeren mit einer tertiären Aminogruppe im Alkylrest zusammen.

Geeignete Monomere mit funktionellen tertiären Aminogruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethyl)propylacrylat, Dimethylamino-2,2-dimethyl)propylmethacrylat, (3-Diethylamino-2,2-dimethyl)propylacrylat und Diethylamino-2,2-dimethyl)propylmethacrylat. Besonders bevorzugt ist Dimethylaminoethylmethacrylat.

Der Gehalt der Monomere mit tertiären Aminogruppen im Copolymeren kann vorteilhafterweise zwischen 20 und 70 Gew.-%, bevorzugt zwischen 40 und 60 Gew.% liegen. Der Anteile der C₁- bis C₄-Alkylestern der Acryl- oder Methacrylsäure beträgt 80 - 30 Gew.-%. Zu nennen sind Methylmethacrylat, Ethylmethacrylat, Butylmethacrylat, Methylacrylat, Ethylacrylat und Butylacrylat.

Ein geeignetes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein.

Ein konkret geeignetes handelsübliches (Meth)acrylatcopolymer mit tertiären Aminogruppen ist z. B. aus 25 Gew.-% Methylmethacrylat, 25 Gew.-% Butylmethacrylat und 50 Gew.-% Dimethylaminoethylmethacrylat aufgebaut (EUDRAGIT® E100).

### Typ EUDRAGIT® RS/RL

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungsverfahren ist die Substanzpolymerisation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Substanzpolymerisation durch Mahlen, bei Lösungs- und Fällungspolymerisation z. B. durch Sprühtrocknung erreichbar ist.

Das (Meth)acrylat-Copolymer, setzt sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid (EUDRAGIT® RS).

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-% 2-Trimethylammoniumethylmethacrylat-Chlorid (EUDRAGIT® RL).

### Dispersion

Das kationische (Methacrylat-)Copolymer liegt in Form einer wässrigen Dispersion, z. B. mit einem Festgehalt von 10 bis 60, bevorzugt 20 bis 40 Gew.-% vor.

Zur Realisierung spezieller Freigabeprofile können in der Dispersion Mischungen der genannten kationisches Methacrylat-Copolymere vorliegen. Die Mischung kann durch Mischen von Pulvern und deren anschließender Überführung in eine Mischdispersion oder durch Mischen einzelner Dispersionen im wässrigen Zustand erfolgen.

Die Viskosität der Polymerdispersion ist bevorzugt etwa gleich oder besonders bevorzugt höher als die Viskosität der wässrigen Polymerlösung, die als Fällungsbad eingesetzt wird. Die Viskosität der Polymerdispersion, gemessen nach mit einem Brookfield Rotationsviskosimeter nach Pharm. Eur. 3^{rd} Edition (1997), Methode 2.2.10. (Rotating Viscometer Method) kann z. B. 20 bis 5000 mPas betragen.

Die Polymerdispersion kann auf eine Temperatur unterhalb der Raumtemperatur eingestellt werden.

### Pharmazeutischer Wirkstoff

Die Dispersion enthält einem oder mehrere pharmazeutische Wirkstoffe in gelöster oder dispergierter Form. Der Wirkstoff kann z. B. zunächst in Wasser oder einem anderen geeigneten Lösungsmittel gelöst bzw. dispergiert werden und dann zu der Dispersion mit dem kationischen Methacrylat-Copolymer gegeben werden, bzw, dort eingemischt werden.

### Pharmazeutische Wirkstoffe

Die erfindungsgemäße Formulierung eignet sich zur Verabreichung grundsätzlich beliebiger pharmazeutischer Wirkstoffe, die vorzugsweise im Darm und/oder Colon freigesetzt werden sollen, und insbesondere solcher, die mit Vorteil in retardierter Form verabreicht werden können, wie Antidepressiva, Betarezeptorenblocker, Antidiabetika, Analgetika, Antiphlogistika, Antirheumatika,Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen-Darmtherapeutika, Migränemittel, Mineralstoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, , Nichtsteroidale Antirheumatika, Herzglykoside, Acetylsalicylsäure, Virustatika, Aclarubicin, Acyclovir, Cisplatin, Actinomycin, alpha- und beta-Sympatomimetika, (Allopurinol, Alosetron, Alprostadil, Prostaglandine, Amantadin, Ambroxol, Amlodipin, Methotrexat, S-Aminosa-licylsäure, Amitriptylin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridarnoi, Domperidon und Domperidanderivate, Donepzil, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolderivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Anti6strogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zoplicon, Zotepin und dergleichen.

Beispiele besonders bevorzugter Wirkstoffe sind Analgetika, wie Tramadol oder Morphin, Mittel zur Behandlung von Colitis ulcerosa oder Morbus Crohn, wie 5-Aminosalicylsäure, Corticosteroide, wie Budesonid, Protonenpumpeninhibitoren, wie Omeprazol, Virusstatika, wie Acyclovir, Lipidsenker, wie Simvastatin oder Pravastatin, H2-Blocker, wie Ranitidin oder Famotidin, Antibiotika, wie Amoxicillin und/oder Clavulansäure, und ACE-Hemmer, wie Enalapril oder Amlodipin.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

### Hilfsstoffe

Hilfsstoffe die der Tropfflüssigkeit zugesetzt werden können sind z. B. Weichmacher,. Zu nennen sind weiterhin Verdickungsmittel, z. B. feindisperse Kieselsäure, wasserlösliche Cellulosederivate, Acrylate, Polyvinylpyrolidon-Typen und Lösungsvermittler wie z. B. Polyethylenglykole.

### Verfahrensschritt b)

Zertropfen der Dispersion, enthaltend den pharmazeutischen Wirkstoff und das Copolymer in eine wässrige Polymerlösung, die ein mit dem kationischen Copolymer unverträgliches anionisches Copolymer enthält, wodurch es zu einer Ausfällung wirkstoffhaltiger Pellets kommt

### Zertropfen der Dispersion

Zum Zertropfen der Polymerdispersion wird eine Tropfapparatur eingesetzt wird, die ein Zerteilen eines Flüssigkeitsstrahls der Polymerdispersion bewirkt. Dies kann z. B. ein mechanischer Zerhacker, eine rotierende Lochscheibe oder eine vibrierende Düse sein. Geeignete Apparaturen sind z. B. auch in Graf, E. und Bothe W "Mikroverkapselung durch Zertropfen". (Pharmazie in unserer Zeit, 1984, Nr. 3, S. 71 bis 82) beschrieben.

Eine entsprechende Apparatur kann z. B. so ausgelegt sein, daß ein Frequenzgenerator einen Vibrator ansteuert, der eine Düse in axiale Schwingungen versetzt. Der Düseninnendurchmesser kann z. B. 0,1 bis 0,5 mm betragen. Die Frequenz der Schwingungen kann beispielweise im Bereich von 5 Hz bis 5 kHz, bevorzugt von 20 bis 100 Hz liegen, wobei Amplitude im Bereich von 0 bis 3 mm stufenlos regulierbar ist.

Die Überwachung des Tropfenbildungsprozesses kann erfolgen, indem die Tropfenbildungsfrequenz gemessen wird. Daraufhin kann die Frequenz der sogenannten Störschwingung so eingestellt werden, daß der Flüssigkeitsstrahl in Resonanz schwingt. Für die Überwachung des Strahlzerfalls kann ein Lichtmessverfahren eingesetzt werden, mit dem sowohl die Tropfenbildungsfrequenz als auch die Größe der Tropfen gemessen werden können.

Die Tropfenbildungsfrequenz kann mit einem Frequenzmesser als Zahl elektrischer Impulse pro Zeiteinheit gemessen werden. Sind die Tropfenbildungsfrequenz und die Frequenz der Störschwingung gleich groß, so schwingt der Flüssigkeitsstrahl in Resonanz und es bilden sich gleich große Tropfen. Die Signale können auf einem Oszilloskop dargestellt werden oder mit Hilfe von Lichtschranken oder Stroboskopen überwacht werden.

Das System kann über einen Mess- und Regelkreis gesteuert werden, der bei Abweichungen von vorgegebenen Soll-Werten entsprechende Korrekturen automatisch vornimmt und die Ist-Werte so innerhalb bestimmter Toleranzen hält.
Erfindungsgemäß können für den Prozess auch konzentrische Mehrfachdüsen eingesetzt werden, bei denen sich die innere Tropfflüssigkeit von der äußeren unterscheidet. In diesem Fall muß nur die äußere Tropfflüssigkeit mit dem Polymer im Fällungsbad unverträglich sein. Es entstehen ebenfalls Pellets mit Kern/Schale-Aufbau.

Ein weiteres zum Zertropfen der Flüssigkeit geeignetes Prinzip ist das JETCUTTING® Verfahren der Firma GeniaLab® Biotechnologie Produkte und Dienstleistungen GmbH, D-38116 Braunschweig,

### Wässrige Polymerlösung

Die wässrige Polymerlösung, enthält ein mit dem kationischen Copolymer unverträgliches anionisches Copolymer. Die wässrige Polymerlösung kann vereinfacht auch als Fällungsbad bezeichnet werden.

Das anionische -Copolymer besteht bevorzugt aus radikalisch polymerisierten Einheiten von C₁- bis C₄- Alkylestern der Acryl- oder Methacrylsäure und Einheiten von (Meth)acrylat-Monomeren mit Carboxylgruppenresten.

Das (Meth)acrylat-Copolymere besteht zu 25 bis 95, bevorzugt zu 45 bis 90, insbesondere zu 45 bis 55 Gew.-% aus radikalisch polymerisierten C₁- bis C₄-Alkylestern der Acryl- oder der Methacrylsäure und kann 5 bis 75, bevorzugt 10 bis 60, insbesondere 40 bis 60 Gew.-% (Meth)acrylat-Monomere mit einer anionischen Gruppe im Alkylrest enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

Geeignet sind anionische (Meth)acrylat-Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Ebenso geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

Weiterhin geeignet sind anionische Vinylcopolymere und Cellulosederivate, wie Crotonsäure-Copolymere, Polyvinylacetatphthalat (PVAP), Celluloseacetatphthalat (CAP), Celluloseacetatsuccinat (CAS), Celluloseactetattrimellitat (CAT), Hydroxypropylmethylcellulosephthalat (HPMCP) und/oder Carboxymethylcellulose (CMEC).

Erfindungsgemäß können auch Mischungen dieser Polymere in Fällungsbad eingesetzt werden.

Um eine Lösung des anionischen Copolymers herzustetten ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH. Es ist auch möglich ein Pulver eines anionischen Copolymeren einzusetzen, dem bereits bei seiner Herstellung zum Zweck der (Teil)neutralisation eine Base z. B. NaOH zugesetzt wurde, so daß das Pulver ein bereits (teil)neutralisierte Polymer ist. Der pH-Wert der Lösung liegt in der Regel über 4.

Die Viskosität der wässrigen Polymerlösung ist bevorzugt etwa gleich oder besonders bevorzugt niedriger als die Viskosität der Polymerdispersion und kann z. B. 10 bis 1000 mPa s (gemessen nach DIN/ISO ?) betragen.

Die wässrige Polymerlösung kann auf eine Temperatur unterhalb der Raumtemperatur z. B. 5, 10 oder 20 °C niedriger eingestellt werden. Die Temperatur der wässrigen Polymerlösung kann z. B. 4 bis 25 °C betragen. Eine niedrige Temperatur ist günstig um Agglomerationen zu vermeiden.

### Hilfsstoffe

Hilfsstoffe die dem Fällungsbad zugesetzt werden können sind z. B. osmotisch wirksame Elektrolyte, die das Aushärten der sphärisch coagulierten Partikel beschleunigen und damit eine gleichmäßige Ausformung fördern. Zu nennen sind weiterhin Trennmittel, die ein Verkleben der Pellets verhindern. Beispiele sind lipophile Emulgatoren mit einem HLB-Wert unter 7 oder übliche Formtrennmittel, wie Silikone, Talkum, Magnesiumstearat, gemahlene Kieselsäure oder Kaolin.

### Verfahrensschritt c)

Abtrennen der wirkstoffhaltigen Pellets aus der wässrigen Polymerlösung und anschließendes Trocknen der Pellets.

Die ausgefällten Pellets können z. B. über Siebe, zweckmäßigerweise wechselbare Siebe aufgefangen werden. Die Trocknung kann nach üblichen Verfahren z. B. in Horden oder in der Wirbelschicht eines Wirbelschichtgenerators erfolgen. Dabei soll die Temperatur unterhalb der Glastemperatur Tg der verwendeten Methacrylat-Copolymere liegen, um ein Verkleben der Pellets zu vermeiden. Die Trocknung kann z. B. für 24 bis 72 Stunden bei 30 bis 50 °C in einem Umlufttrockenschrank erfolgen.

### Wirkstoffhaltige Pellets mit mehrschichtigem Kern/Schale-Aufbau

Die nach dem erfindungsgemäßen Verfahren erhältlichen wirkstoffhaltige Pellets weisen einen mehrschichtigen Kern/Schale-Aufbau auf. Dabei ist der Wirkstoff im Kern zusammen mit dem kationischen Copolymer formuliert, es schließt sich eine Übergangszone an in der eine übergangsweise, gleitende Zone von Wirkstoff kationischen Copolymer und damit vermengtem anionischen Copolymer anschließt und schließlich von einer äußeren Schale mit dem anionischen Copolymer umschlossen ist.

Der Wirkstoffgehalt nimmt von Kern her in der Übergangszone ab und kann dabei z. B. übergangsweise von der partikulären Form (solid dispersion) in eine gelöste Form (solid solution) mit höherer Bioverfügbarkeit übergehen. Die Unterscheidung bzw. der Nachweis der beiden Zustandsformen kann z. B. durch DSC (Differential Scanning Calorimetrie) oder mittels Röntgenbeugungsanalyse erfolgen.

Die Partikel können rund oder leicht elipsoid sein und weisen Größen bzw. Durchmesser im Bereich von 100 bis 2000 µm auf.

Im Vergleich zu einem konventionellen zweischichtigen Aufbau von wirkstoffhaltigen Pellets wie er z. B. aus DE 100 13 029 bekannt ist, können andere, verzögerte Freigabeprofile erhalten werden, zu denen einerseits die Partikelgröße, aber insbesondere die Übergangszone beiträgt. Weitere vorteilhafte Eigenschaften der erfindungsgemäßen Pellets sind Geschmacks- und Geruchsisolierung oder verbesserte Bioverfügbarkeit schwerlöslicher Wirkstoffe.

### Arzneiformen

Die erfindungsgemäß hergestellten Pellets können zu Arzneiformen verarbeitet werden, durch Vertüllen in Kapseln oder Sachets, durch Verpressen zu Tabletten mit beschleunigtem oder verzögertem Zerfall, als Bestandteil von Trockensäften, als Basismaterial für Einbettungen in weitere Arzneiformen, wie Suppositorien, Vaginalia, Implantaten, Filmen oder Dermatika, wie z. B. transdermalen Therapiesystemen. Als weitere Anwendungsgebiete sind Diagnostika oder Kosmetika zu nennen.

### BEISPIELE

EUDRAGIT® RS 30D : 30%-ige Dispersion enthaltend ein Copolymer aus 65 Gew.-% Methylmethacrylat, 30 Gew.-% Ethylacrylat und 5 Gew.-% 2-Trimethylammoniumethylmethacrylat-Chlorid.
EUDRAGIT® L 100 - 55: Copolymer aus 50 Gew.-% Methacrylsäure und 50 Gew.-% Ethylacrylat.

### Beispiel 1: Retardierte Propranolol Pellets

### a) Tropfdispersion:

| **Rezeptur** | **Gew.-%** | **Trockensubstanz (Gew.-%)** | **Gew.-% in der Trockensubstanz** |
|---|---|---|---|
| EUDRAGIT^{®} RS 30 D | 89.0 | 26.7 | 71.8 |
| Polysorbat 80 | 0.4 | 0.4 | 1.1 |
| Acetyltributylcitrat | 4.0 | 4.0 | 10.8 |
| HCl (10%ig) | 0.7 | 0.1 | 0.2 |
| Propranolol - HCl | 6.0 | 6.0 | 16.1 |
| Summe | 100.0 | 37.2 | 100.0 |

Polysorbat 80 und Acetyltributylcitrat werden mit einem Rührwerk (z.B. Magnetrührer homogenisiert. Die Einarbeitung des EUDRAGIT RS 30 D erfolgt mit einem schnelllaufenden Rührwerk (z. B. Ultra Turrax). Mit 10%iger HCl wird die Dispersion auf pH 3,5 eingestellt und abschließend, nach Zugabe von Propranolol HCl, weitere 10 min. mit dem Ultra Turrax homogenisiert.

### b) Fällungsbad

| **Rezeptur** | **Gew.-%** | **Trockensubstanz** | **Gew.-% in der Trockensubstanz** |
|---|---|---|---|
| **EUDRAGIT^{®} L100-55** | 5.00 | 5.0 | 38.5 |
| | | | |
| | | | |
| Natronlauge 20%ig, wässrig, zu 45% teilneutralisiert mit NaOH | 2.50 | 0.5 | 3.8 |
| NaCl- Lösung 20%ig, | | 7.5 | 57.7 |
| | | | |
| wässrig | 37.50 | | |
| | | | |
| Wasser, gereinigt | 55.00 | -- | -- |
| Summe | 100.00 | 13.0 | 100.0 |

In das Wasser wird EUDRAGIT L 100-55 (Zugabe portionsweise) mit einem Flügelrührer dispergiert. Nach Zugabe der Natronlauge und der NaCl - Lösung wird die entstandene Lösung 1 Stunde nachgerührt.

### c) Tropfbedingungen:

V=148 g Dispersion/Std. (Förderung: Exzenterschneckenpumpe)
Schwingungsfrequenz an der Düse beim Zertropfvorgang: 50 Hz Düseninnendurchmesser: 0.3 mm

### d) Separation und Trocknung

Separation über Filtervorrichtung (siehe Zeichnung).

Trocknung 48 Stunden 40°C (Umlufttrockenschrank)

### e) Daten der Pellets:

Form: rund bis leicht elipsoid, fest, 62 % Wirkstoffgehalt (UV-photometrisch analysiert)

| Siebanalyse: | |
|---|---|
| >2.0 mm : | 2.3% (Masse) |
| 2.0-1.4 mm : | 30.1 % |
| 1.4-1.0 mm: | 57.1 % |
| 1.0-0.8mm : | 9.8 % |
| 0.8-0.5 mm | 0.4 % |
| <0.5 mm : | 0.3 % |

### Analysenwerte :

Wirkstofffreigabe (Paddle 100 Upm) 2 Std. pH 1,0 danach pH 6.8 umgepuffert:
Wirkstoffgehalt: 10.0 %(=62.0 % der Theorie)

| [Zeit Std.] | % Freigabe der Theorie |
|---|---|
| 0.5 | 59.7 |
| 1 | 52.5 |
| 2 | 96.0 |
| 3 | 96.1 |
| 4 | 98.9 |
| 6 | 99.4 |

### Beispiel 2: Retardierte Verapamil Pellets (VB 89/55)

### a) Tropfdispersion:

| **Rezeptur** | **Gew.-%** | **Trockensubstanz (Gew.-%)** | **Gew.-% in der Trockensubstanz** |
|---|---|---|---|
| EUDRAGIT^{®} RS 30 D | 91.8 | 27.5 | 77.0 |
| Polysorbat 80 | 1.8 | 1.8 | 5.1 |
| Acetyltributylcitrat | 3.0 | 3.0 | 8.4 |
| HCl (10%ig) | 0.7 | 0.1 | 0.3 |
| Verapamil- HCl | 3.3 | 3.3 | 9.2 |
| Summe | 100.0 | 35.7 | 100.0 |

Polysorbat 80 und Acetyltributylcitrat werden mit einem Rührwerk (z.B. Magnetrührer homogenisiert. Die Einarbeitung des EUDRAGIT RS 30 D erfolgt mit einem schnelllaufenden Rührwerk (z. B. Ultra Turrax). Mit 10%iger HCl wird die Dispersion auf pH 3,5 eingestellt und abschließend, nach Zugabe von Verapamil- HCl, weitere 10 min. mit dem Ultra Turrax homogenisiert.

### b) Fällungsbad analog Beispiel 1

### c) Tropfbedingungen:

V=239 g Dispersion/Std. (Förderung: Exzenterschneckenpumpe) Schwingungsfrequenz an der Düse beim Zertropfvorgang: 50 Hz Düseninnendurchmesser: 0.3 mm

### d) Separation und Trocknung

Separation über Filtervorrichtung (siehe Zeichnung).
Trocknung 48 Stunden 40°C (Umlufttrockenschrank)

### e) Daten der Pellets:

Form: rund bis leicht elipsoid, fest,

| Siebanalyse: | |
|---|---|
| >2.0 mm : | 19.4% (Masse) |
| 2.0-1.0 mm: | 52.2% |
| 1.0-0.5 mm: | 26.9% |
| <0.5 mm : | 1.5% |

### Analysenwerte :

Wirkstoffgehalt: 7,44 %(=80,6 % der Theorie)

Wirkstofffreigabe (Matrixkugeln 1.0-2.0 mm; Paddle 100 Upm) 2 Std. pH 1,0 danach pH 6.8 umgepuffert:

| [Zeit Std.] | % Freigabe der 0.50-1.00 mm Fraktion | % Freigabe der 1.00-2.00 mm Fraktion | % Freigabe der 2.00-3.2 mm Fraktion |
|---|---|---|---|
| 0 | 0 | 0 | 0 |
| 2 | 94 | 75.9 | 53 |
| 3 | 95 | 82.0 | 56 |
| 4 | 95.8 | 83.3 | 57 |
| 6 | 96.5 | 87.1 | 58 |
| 8 | 99 | 88.2 | 61 |
| Homogenisiert | 100 | 100 | 100 |

### Beispiel 3: Retardierte Propranolol Pellets mit verdickter Tauchdispersion

### a) Tropfdispersion:

| **Rezeptur** | **Gew.-%** | **Trockensubstanz (Gew.-%)** | **Gew.-% in der Trockensubstanz** |
|---|---|---|---|
| EUDRAGIT^{®} RS 30 D | 92.0 | 27.6 | 77.5 |
| | | | |
| Propranolol HCl | 6.2 | 6.2 | 17.4 |
| | | | |
| Aerosil^{®} 200 | 1.8 | 1.8 | 5.1 |
| | | | |
| Summe | 100.0 | 35.6 | 100.0 |

EUDRAGIT RS 30 D wird vorgelegt und nacheinander Propranolol HCl und Aerosil 200 mit dem Ultra Turrax in die Dispersion dispergiert (Dispergierzeit jeweils 10 Minuten).

### b) Fällungsbad analog Beispiel 1

### c) Tropfbedingungen:

V=283 g Dispersion/Std. (Förderung: Exzenterschneckenpumpe) Schwingungsfrequenz an der Düse beim Zertropfvorgang: 50 Hz Düseninnendurchmesser: 0.3 mm

### d) Separation und Trocknung

Separation über Filtervorrichtung (siehe Zeichnung).
Trocknung 48 Stunden 40°C (Umlufttrockenschrank)

**e) Daten der Pellets**: Form: rund bis leicht elipsoid, fest.

## Patentansprüche

1. Verfahren zur Herstellung von wirkstoffhaltigen Pellets
durch die Schritte
a) Suspendieren oder Lösen eines pharmazeutischen Wirkstoffs in einer Dispersion, die ein kationisches Copolymer enthält
b) Zertropfen der Dispersion, enthaltend den pharmazeutischen Wirkstoff und das Copolymer in eine wässrige Polymerlösung, die ein mit dem kationischen Copolymer unverträgliches anionisches Copolymer enthält, wodurch es zu einer Ausfällung wirkstoffhaltiger Pellets kommt
c) Abtrennen der wirkstoffhaltigen Pellets aus der wässrigen Polymerlösung und anschließendes Trocknen der Pellets

2. Verfahren nach Anspruch 1, **dadurch gekennzeichnet, dass** das kationische Copolymer aus radikalisch polymerisierten Einheiten von C₁-bis C₄- Alkylestern der Acryl- oder Methacrylsäure und Einheiten von (Meth)acrylat-Monomeren mit tertiären oder quaternären Amino- bzw. Ammoniumgruppen besteht.

3. Verfahren nach Anspruch 1 oder 2, **dadurch gekennzeichnet, dass** das anionische Copolymer aus radikalisch polymerisierten Einheiten von C₁-bis C₄- Alkylestern der Acryl- oder Methacrylsäure und Einheiten von (Meth)acrylat-Monomeren mit Carboxylgruppenresten besteht.

4. Verfahren nach einem oder mehreren der Ansprüche 1 bis 3, **dadurch gekennzeichnet, dass** die Viskosität der Polymerdispersion höher ist als die Viskosität der wässrigen Polymerlösung.

5. Verfahren nach Anspruch 4, **dadurch gekennzeichnet, dass** die Viskosität der Polymerdispersion, gemessen nach Pharm. Eur. 3^{rd} Edition, Methode 2.2.10, 20 bis 5000 mPa s und die Viskosität der wässrigen Polymerlösung 10 bis 1000 mPa s beträgt.

6. Verfahren nach einem oder mehreren der Ansprüche 1 bis 5, **dadurch gekennzeichnet, dass** die Polymerdispersion und/oder die wässrige Polymerlösung auf eine Temperatur unterhalb der Raumtemperatur eingestellt werden.

7. Verfahren nach einem oder mehreren der Ansprüche 1 bis 6, **dadurch gekennzeichnet, dass** zum Zertropfen der Polymerdispersion eine Tropfapparatur eingesetzt wird, die ein Zerteilen eines Flüssigkeitsstrahls der Polymerdispersion bewirkt.

8. Wirkstoffhaltige Pellets mit mehrschichtigem Kern/Schale-Aufbau herstellbar in einem Verfahren nach einem oder mehreren der Ansprüche 1 bis 7.

9. Arzneiform, **dadurch gekennzeichnet, dass** Pellets nach Anspruch 8 enthalten sind.

10. Arzneiform nach Anspruch 9, **dadurch gekennzeichnet, dass** es sich um Kapseln, Sachets, Tabletten mit beschleunigtem oder verzögertem Zerfall, Trockensäfte, Suppositorien, Vaginalia, Implantate, Filme oder Dermatika, wie z. B. transdermalen Therapiesystemen handelt.

11. Verwendung wirkstoffhaltiger Pellets gemäß Anspruch 8 zur Herstellung von Diagnostika oder Kosmetika.

## Claims

1. Method for the production of active ingredient-containing pellets
by the steps of
a) suspending or dissolving an active pharmaceutical ingredient in a dispersion which comprises a cationic copolymer
b) delivering droplets of the dispersion comprising the active pharmaceutical ingredient and the copolymer into an aqueous polymer solution which comprises an anionic copolymer incompatible with the cationic copolymer, resulting in precipitation of active ingredient-containing pellets
c) removal of the active ingredient-containing pellets from the aqueous polymer solution and subsequent drying of the pellets.

2. Method according to Claim 1, **characterized in that** the cationic copolymer consists of free-radical polymerized units of C₁- to C₄-alkyl esters of acrylic or methacrylic acid and units of (meth)acrylate monomers with tertiary or quaternary amino or ammonium groups.

3. Method according to Claim 1 or 2, **characterized in that** the anionic copolymer consists of free-radical polymerized units of C₁- to C₄-alkyl esters of acrylic or methacrylic acid and units of (meth)acrylate monomers with carboxyl group radicals.

4. Method according to one or more of Claims 1 to 3, **characterized in that** the viscosity of the polymer dispersion is higher than the viscosity of the aqueous polymer solution.

5. Method according to Claim 4, **characterized in that** the viscosity of the polymer dispersion measured by method 2.2.10 to Pharm. Eur. 3^{rd} edition is from 20 to 5000 mPa s and the viscosity of the aqueous polymer solution is from 10 to 1000 mPa s.

6. Method according to one or more of Claims 1 to 5, **characterized in that** the polymer dispersion and/or the aqueous polymer solution are adjusted to a temperature below room temperature.

7. Method according to one or more of Claims 1 to 6, **characterized in that** a dropping apparatus which breaks up a liquid jet of the polymer dispersion is employed for the delivery of droplets of the polymer dispersion.

8. Active ingredient-containing pellets with a multilayer core/shell structure which can be produced in a method according to one or more of Claims 1 to 7.

9. Pharmaceutical form, **characterized by** a content of pellets according to Claim 8.

10. Pharmaceutical form according to Claim 9,
**characterized in that** it comprises capsules, sachets, tablets with accelerated or delayed disintegration, powders for reconstitution, suppositories, products for vaginal use, implants, films or dermatologicals such as, for example, transdermal therapeutic systems.

11. Use of active ingredient-containing pellets according to Claim 8 for producing diagnostic aids or cosmetics.

## Revendications

1. Procédé de fabrication de pastilles contenant une substance active grâce aux étapes consistant à :
a) mettre en suspension ou dissoudre une substance active pharmaceutique dans une dispersion qui contient un copolymère cationique,
b) scinder goutte par goutte la dispersion contenant la substance active pharmaceutique et le copolymère dans une solution polymère aqueuse qui contient un copolymère anionique incompatible avec le copolymère cationique, grâce à quoi se produit une précipitation de pastilles contenant de la substance active
c) séparer les pastilles contenant de la substance active issues de la solution polymère aqueuse et sécher ensuite les pastilles.

2. Procédé selon la revendication 1, **caractérisé en ce que** le copolymère cationique est constitué d'unités polymérisées par voie radicalaire d'esters alkyliques en C₁ à C₄ de l'acide acrylique ou de l'acide méthacrylique et d'unités de monomères de (méth)acrylate ayant des groupes amino ou ammonium tertiaires ou quaternaires.

3. Procédé selon la revendication 1 ou 2, **caractérisé en ce que** le copolymère anionique est constitué d'unités polymérisées par voie radicalaire d'esters alkyliques en C₁ à C₄ de l'acide acrylique ou de l'acide méthacrylique et d'unités de monomères de (méth)acrylate ayant des groupes radicaux carboxyle.

4. Procédé selon une ou plusieurs des revendications 1 à 3, **caractérisé en ce que** la viscosité de la dispersion polymère est plus élevée que la viscosité de la solution polymère aqueuse.

5. Procédé selon la revendication 4, **caractérisé en ce que** la viscosité de la dispersion polymère, mesurée d'après Pharm. Eur. 3^{e} édition, Méthode 2.2.10, est de 20 à 5000 mPa s et la viscosité de la solution polymère aqueuse est de 10 à 1000 mPa s.

6. Procédé selon une ou plusieurs des revendications 1 à 5, **caractérisé en ce que** la dispersion polymère et/ou la solution polymère aqueuse est ajustée à une température inférieure à la température ambiante.

7. Procédé selon une ou plusieurs des revendications 1 à 6, **caractérisé en ce qu'**un appareil de goutte à goutte est utilisé pour la scission goutte à goutte de la dispersion polymère, qui provoque une défloculation d'une veine liquide de la dispersion polymère.

8. Pastilles contenant de la substance active que l'on peut fabriquer avec une structure en plusieurs couches noyau/enveloppe par un procédé selon une ou plusieurs des revendications 1 à 7.

9. Forme pharmaceutique, **caractérisée en ce qu'**elle contient des pastilles selon la revendication 8.

10. Forme pharmaceutique selon la revendication 9,
**caractérisée en ce qu'**il s'agit de capsules, de sachets, de tablettes à désintégration accélérée ou retardée, de jus déshydratés, de suppositoires, de produits gynécologiques, d'implants, de films ou de produits dermatologiques, comme par exemple des systèmes thérapeutiques transdermiques.

11. Utilisation de pastilles contenant de la substance active selon la revendication 8 pour la fabrication de produits de diagnostic ou de produits cosmétiques.
